# EUROPEAN PATENT SPECIFICATION

(11) **EP 4 093 025 B1**
(45) Date of publication and mention of the grant of the patent: **17.09.2025**
(21) Application number: 22174398.2
(22) Date of filing: 19.05.2022
(51) Int. Cl.: H04N 7/18, A61B 1/00, A61B 5/00, A61B 6/00, A61B 8/00

(54) **MEDICAL IMAGE PROCESSING SYSTEM**
SYSTEM ZUR VERARBEITUNG MEDIZINISCHER BILDER
SYSTÈME DE TRAITEMENT D'IMAGE MÉDICALE

(30) Priority: 21.05.2021 JP 2021086548
(43) Date of publication of application: 23.11.2022
(73) Proprietor: FUJIFILM Corporation, Tokyo 106-8620 (JP)
(72) Inventor: TERAMURA, Yuichi, Kanagawa (JP)
(74) Representative: Dehns Germany Partnerschaft mbB

(56) References cited:
- WO-A1-2021/090056
- JP-U- 3 209 885
- KR-A- 20190 084 623
- US-A1- 2015 302 605
- US-A1- 2019 290 247

## Description

### BACKGROUND OF THE INVENTION

### 1. Field of the Invention

The present invention relates to a medical image processing system.

### 2. Description of the Related Art

In a case in which an endoscope is inserted into the body of a subject to perform observation, the pharynx is mainly imaged to acquire an image in a swallowing examination. In the swallowing examination by the endoscope, a tip portion of the endoscope inserted from a nasal cavity images the pharynx to observe swallowing in a pharyngeal stage. The swallowing includes an oral stage in which food is transported from the tongue to the pharynx, the pharyngeal stage in which the food is transported from the pharynx to the esophagus, and an esophageal stage in which the food is transported to the stomach through the esophagus. The swallowing ability of the patient can be examined by observing the pharyngeal stage with the endoscope. However, it is preferable to obtain information of swallowing in the oral stage or the esophageal stage as well as swallowing in the pharyngeal stage obtained by imaging the pharynx in order to evaluate and determine swallowing in detail. In addition, the swallowing in the pharyngeal stage can be examined by different methods to understand the details of the swallowing. A swallowing contrast examination is a method that observes the movement of a swallowing food containing a contrast medium with an X-ray fluoroscope and observes, for example, the morphology, function, aspiration, and residual swallowing food of the part related to swallowing. In recent years, observation using an ultrasonography device has also been performed. Ultrasonography is an examination method that does not have the risk of radiation exposure, such as X-ray exposure, can be performed from the body surface, and has less burden on the patient. Examples of the ultrasonography include an examination that observes the esophagus and the airway to obtain information of swallowing in the esophageal stage, an examination that observes the laryngopharynx to observe the swallowing, aspiration and residue of food and beverage, and an examination that observes the morphology of the tongue muscles, muscles around the hyoid bone, or the larynx muscles to evaluate a swallowing movement.

However, since a plurality of examination systems are used, the swallowing examination is performed a plurality of times in a short time, which is a burden on the patient. In addition, the examiner separately checks the results of the swallowing examination by each examination system. As a result, this method is inefficient, and the management of the examination results tends to be complicated. In addition, it takes time to review the results.

JP3209885Y discloses a technique that evaluates a swallowing function using a swallowing examination with an endoscope and an X-ray fluoroscope and displays the acquired endoscopic moving image and X-ray contrast image on the same screen. JP5823535B discloses a technique that performs imaging using medical devices, such as an endoscope, a radiography apparatus, and an ultrasound diagnostic apparatus, and wirelessly transmits each acquired image to the same tablet PC in a case in which a specific diagnosis or treatment is performed.

Further relevant prior-art is WO 2021/090056 A1, which discloses an endoscope system with both a visible light camera device and an ultrasound imaging device. The images from both devices are shown simultaneously, KR 2019 0084623 A which discloses an endoscope capsule with both visible imaging and ultrasound imaging capabilities. Images from both are simultaneously displayed, and US 2015/302605 A1 which discloses synchronizing video signal from two medical imaging devices using either timings or markers.

### SUMMARY OF THE INVENTION

In JP3209885Y, the endoscopic moving image and the X-ray moving image are simultaneously displayed in real time. Therefore, there is a difference in playback timing. In addition, JP3209885Y does not disclose a specific swallowing observation part or the execution of ultrasonography. JP5823535B discloses the diagnosis or treatment in an operating room using a plurality of medical devices, but does not disclose a configuration that in which a swallowing examination is performed and imaging devices can be carried. It is desirable to accurately play back the moving images obtained by imaging swallowing with a plurality of systems at the same time, without a time lag, to accurately observe the swallowing, and to efficiently observe the swallowing, on the basis of the above-mentioned points.

An object of the invention is to provide a medical image processing system that reduces a burden on a patient and enables a doctor to efficiently observe swallowing in a swallowing examination using a plurality of examination systems.

A medical image processing system is provided as defined by claim 1.

### BRIEF DESCRIPTION OF THE DRAWINGS

Fig. 1 is a schematic diagram illustrating a medical image processing system having an endoscope system and an ultrasound system.
Fig. 2 is a diagram illustrating food swallowing stages.
Fig. 3 is a diagram illustrating a part observed by an endoscope and an ultrasonography terminal in a swallowing examination.
Fig. 4 is a block diagram illustrating functions of a processor device. display.

Preferably, the endoscopic video signal and the ultrasound video signal are played back automatically or by an operation of a user.

Preferably, the processor records an imaging time based on a time of the processor in a case in which the endoscopic video signal and the ultrasound video signal are acquired, performs a synchronization process for the endoscopic video signal and the ultrasound video signal on the basis of the imaging time, detects a swallowing timing from the endoscopic video signal, detects a non-swallowing timing from the ultrasound video signal, tags the endoscopic video signal having the same imaging time as the ultrasound video signal at a timing other than the non-swallowing timing among the endoscopic video signals at the swallowing timing, extracts an endoscopic index moving image, tags the ultrasound video signal having the same imaging time as the endoscopic video signal at the swallowing timing among the ultrasound video signals at the timing other than the non-swallowing timing, extracts an ultrasound index moving image, and plays back the endoscopic index moving image and the ultrasound index moving image on the display.

Preferably, the endoscopic index moving image and the ultrasound index moving image are played back automatically or by an operation of a user.

Preferably, the processor detects any one of normal swallowing, aspiration, or unknown swallowing as the swallowing timing from the endoscopic video signal and the ultrasound video signal and tags a frame image in which the swallowing timing has been detected with swallowing timing detection.

Preferably, the processor determines whether or not the swallowing timing is present in the endoscopic video signal, using any one of calculation of an amount of blur between the frame images, calculation of a key point based on the frame image, or a difference between pixel values of the frame images and determines whether or not the swallowing timing is present in the ultrasound video signal using machine learning.

Preferably, the processor analyzes at least one of the endoscopic index moving image or the ultrasound index moving image to specify a type of the swallowing examination.

Preferably, the processor classifies the types of the swallowing examinations according to a type of swallowing examination food and a result of swallowing and gives index numbers to the endoscopic index moving image and the ultrasound index moving image on the basis of the classification.

Preferably, the processor performs imaging at a frame rate of 60 fps in the acquisition of the endoscopic video signal and the ultrasound video signal, and time is counted in centiseconds in the imaging.

Preferably, the processor simultaneously displays the endoscopic video signal and the ultrasound video signal on the display in real time without synchronizing the endoscopic video signal and the ultrasound video signal.

Preferably, the processor receives a fluoroscopic video signal obtained by observing the swallowing examination of the patient with a radiation observation device, instead of the observation with the ultrasonography terminal, and may display the endoscopic video signal and the fluoroscopic video signal on the display.

Preferably, the processor receives a fluoroscopic video signal obtained by observing the swallowing examination of the patient with a radiation observation device, instead of the observation with the ultrasonography terminal, and may display the endoscopic video signal, the ultrasound video signal, and the fluoroscopic video signal on the display.

Since the swallowing examinations are performed at the same time using a plurality of swallowing examination systems, the burden on the patient is reduced. Since the acquired moving images are displayed on the same screen, the burden on the doctor is reduced, and swallowing is observed efficiently and accurately.

### BRIEF DESCRIPTION OF THE DRAWINGS

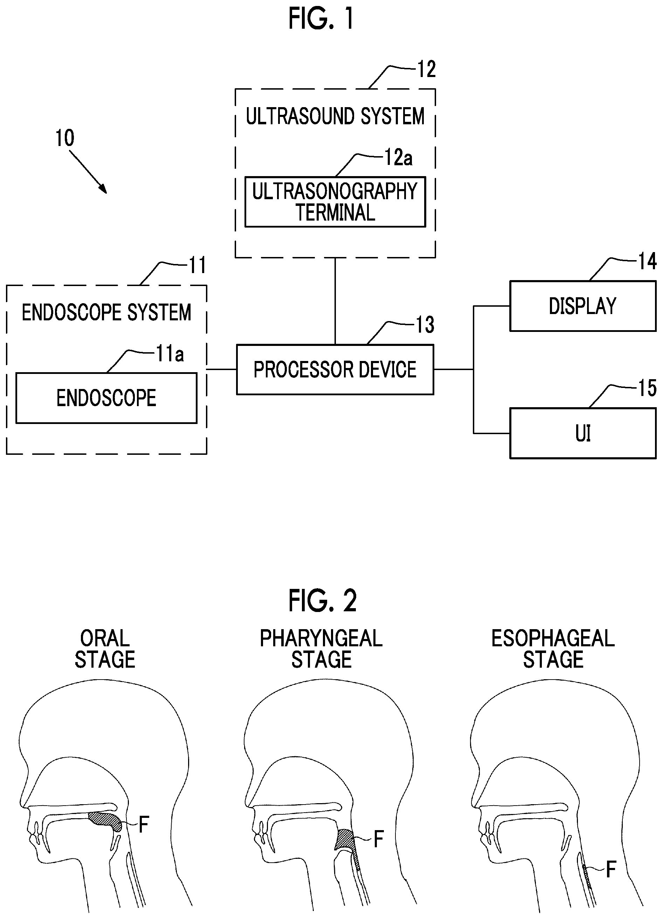

Fig. 1 is a schematic diagram illustrating a medical image processing system having an endoscope system and an ultrasound system.
Fig. 2 is a diagram illustrating food swallowing stages.

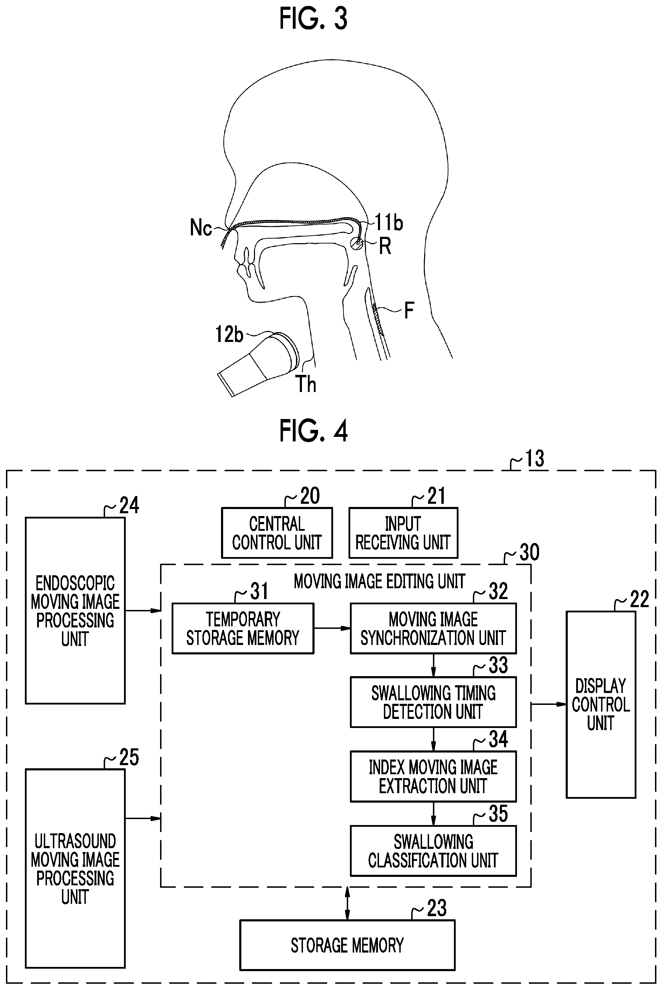

Fig. 3 is a diagram illustrating a part observed by an endoscope and an ultrasonography terminal in a swallowing examination.
Fig. 4 is a block diagram illustrating functions of a processor device.

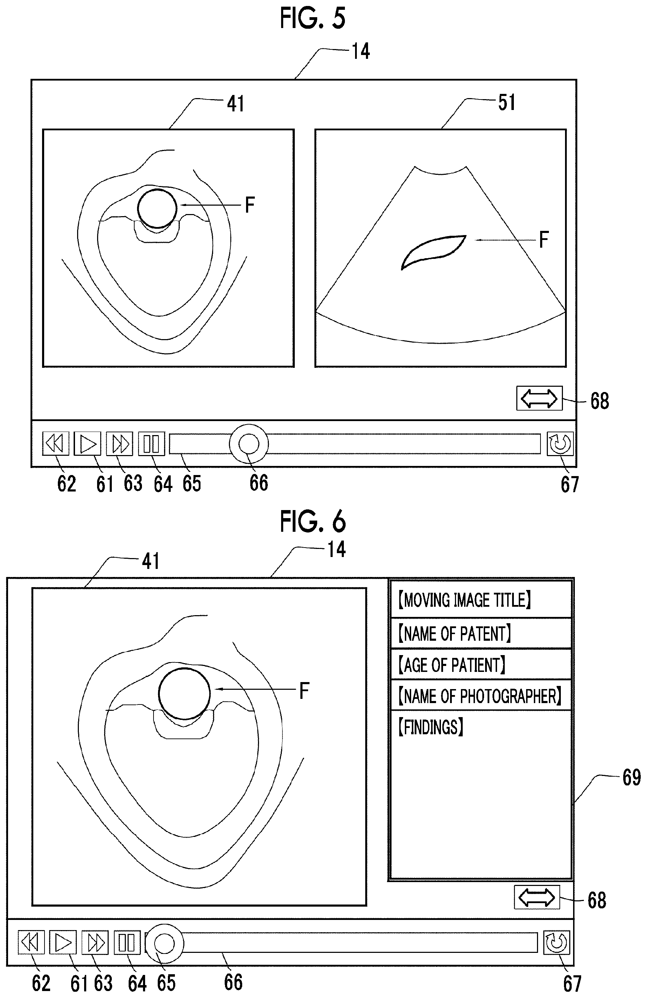

Fig. 5 is a diagram illustrating an example in which an endoscopic moving image and an ultrasound moving image are synchronously displayed on a screen at the same time.
Fig. 6 is a diagram illustrating an example in which the endoscopic moving image is independently displayed on the screen.

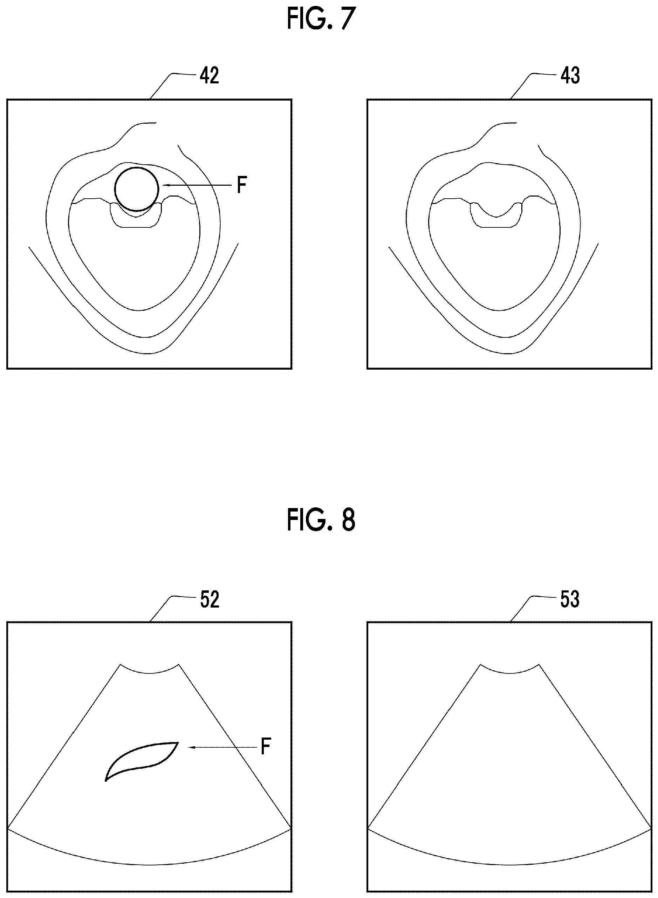

Fig. 7 is a diagram illustrating images of the pharynx in a case in which food is swallowed and in a case in which food is not swallowed.
Fig. 8 is a diagram illustrating images of the esophagus in a case in which food is swallowed and in a case in which food is not swallowed.

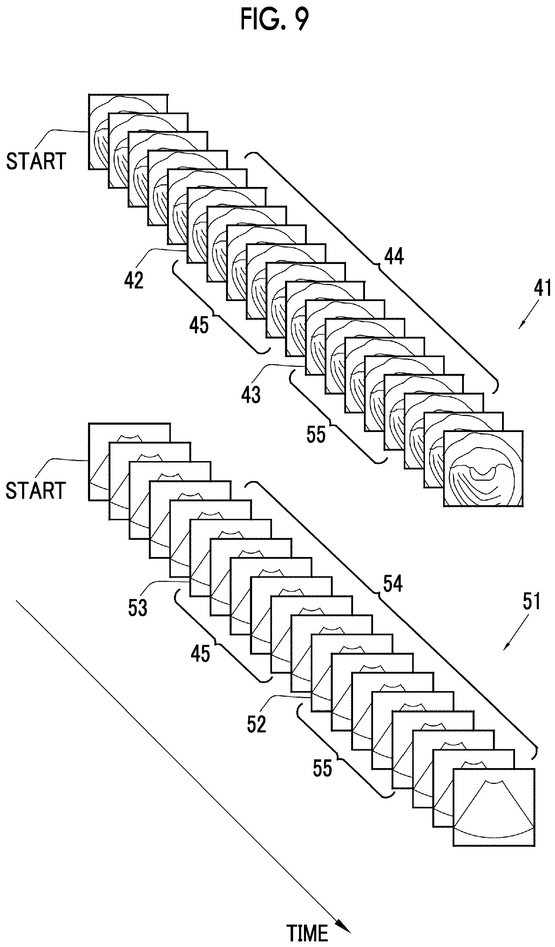

Fig. 9 is a diagram illustrating the extraction of index moving images from the endoscopic moving image and the ultrasound moving image.

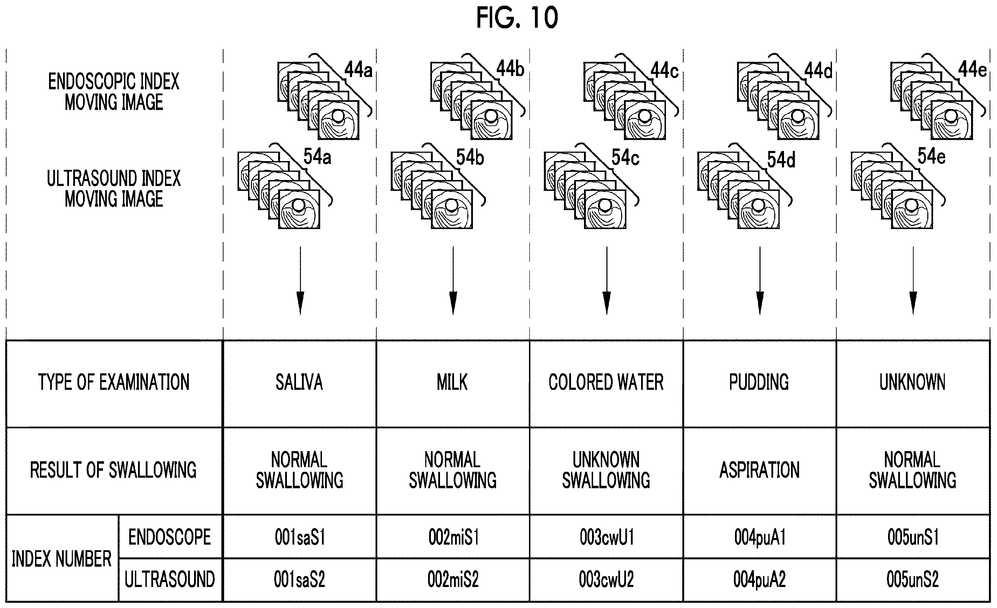

Fig. 10 is a diagram illustrating a classification result of swallowing and the distribution of the classification result to index numbers.

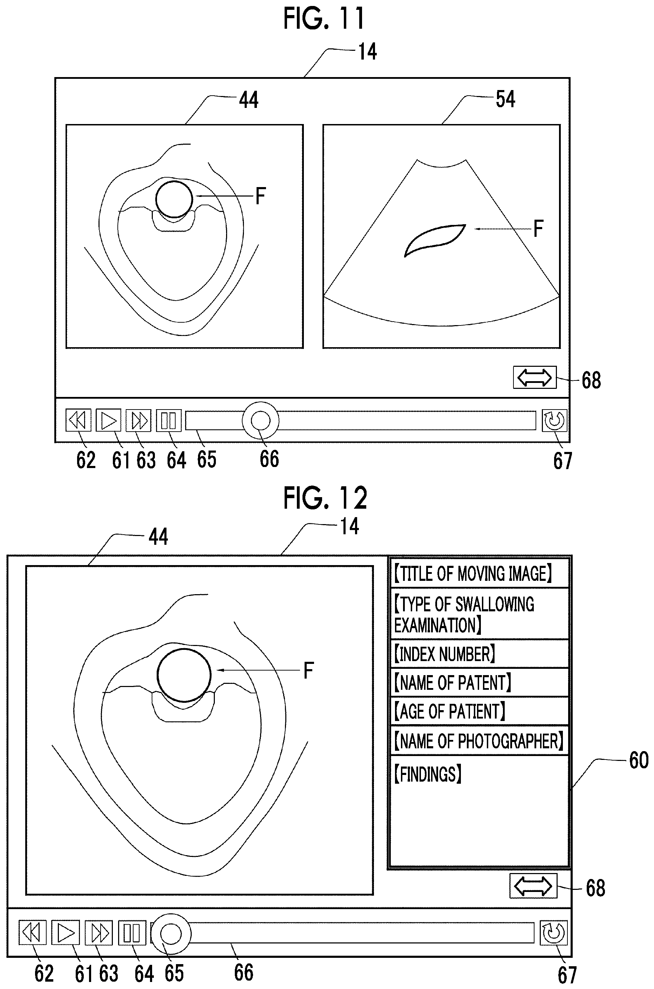

Fig. 11 is an image diagram illustrating an example in which an endoscopic index moving image and an ultrasound index moving image are synchronously played back on the screen at the same time.
Fig. 12 is an image diagram illustrating the playback and display of the endoscopic index moving image.

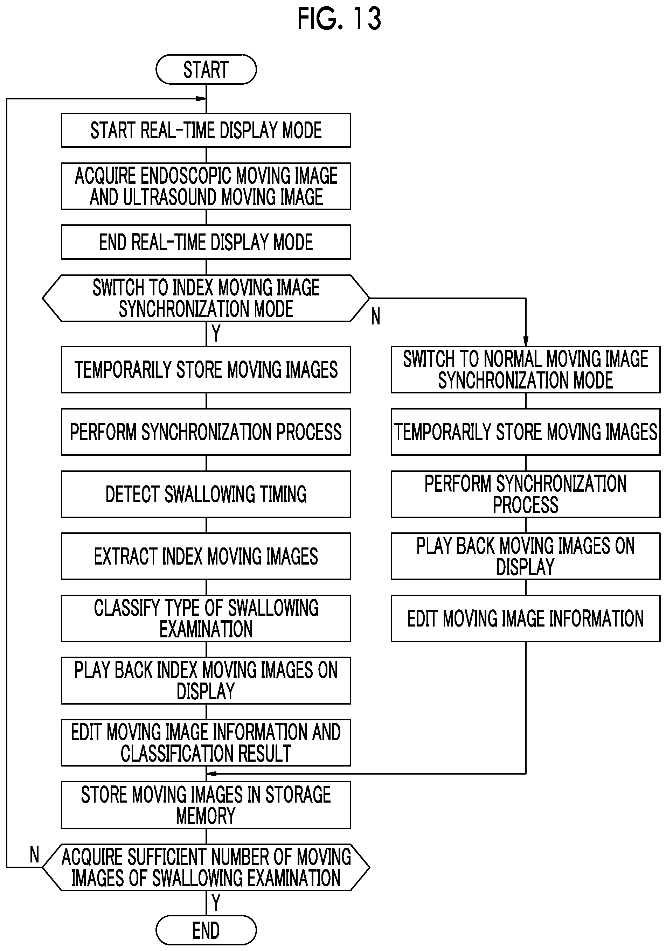

Fig. 13 is a flowchart illustrating the synchronous playback of the endoscopic moving image and the ultrasound moving image.

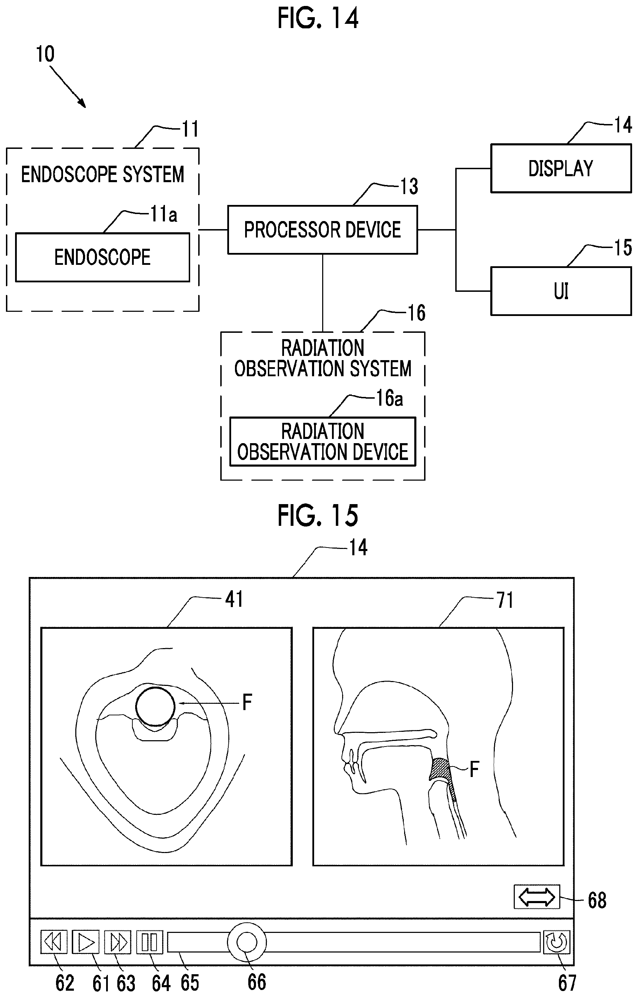

Fig. 14 is a schematic diagram illustrating a medical image processing system having the endoscope system and a radiation observation system according to a second embodiment.
Fig. 15 is an image diagram illustrating the synchronous playback of the endoscopic moving image and a fluoroscopic moving image.

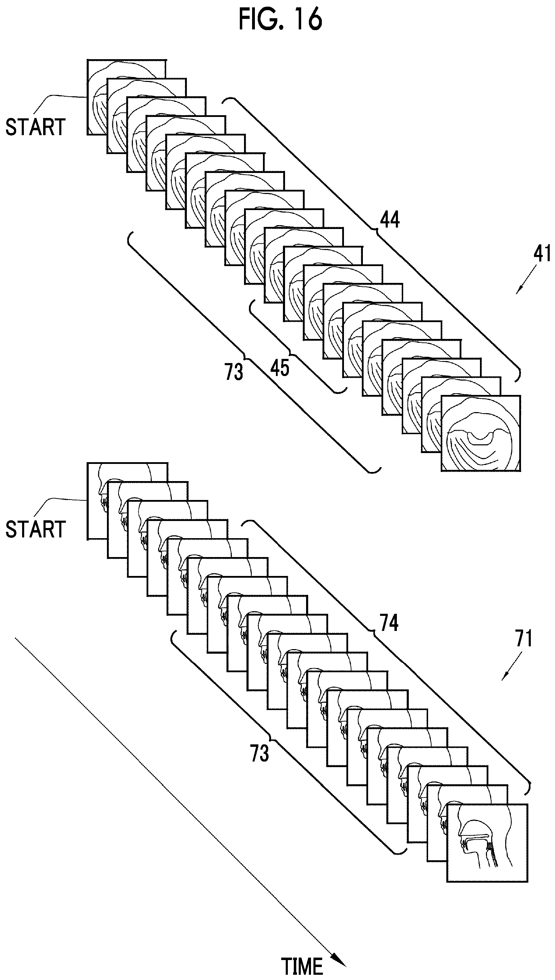

Fig. 16 is an image diagram illustrating the extraction of index moving images from the endoscopic moving image and the fluoroscopic moving image.

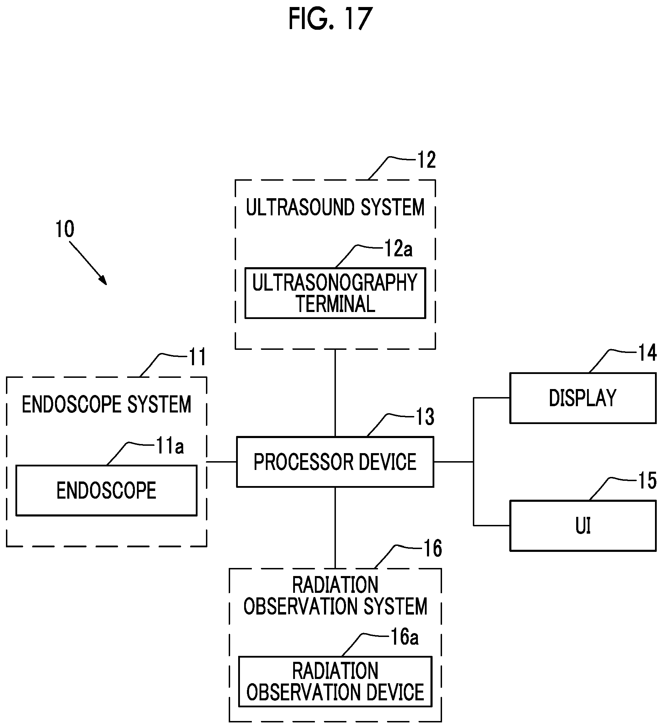

Fig. 17 is a schematic diagram illustrating a medical image processing system having the endoscope system, the ultrasound system, and the radiation observation system according to a third embodiment.

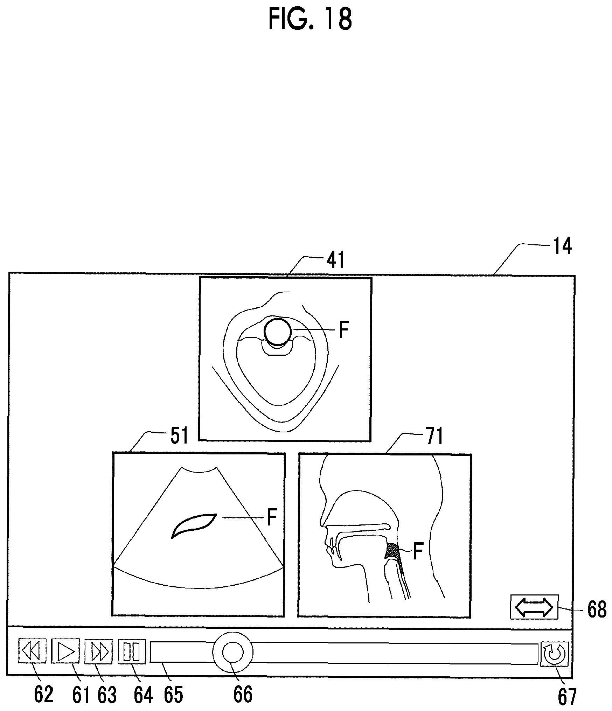

Fig. 18 is an image diagram illustrating the synchronous playback of an endoscopic moving image, an ultrasound moving image, and a fluoroscopic moving image.

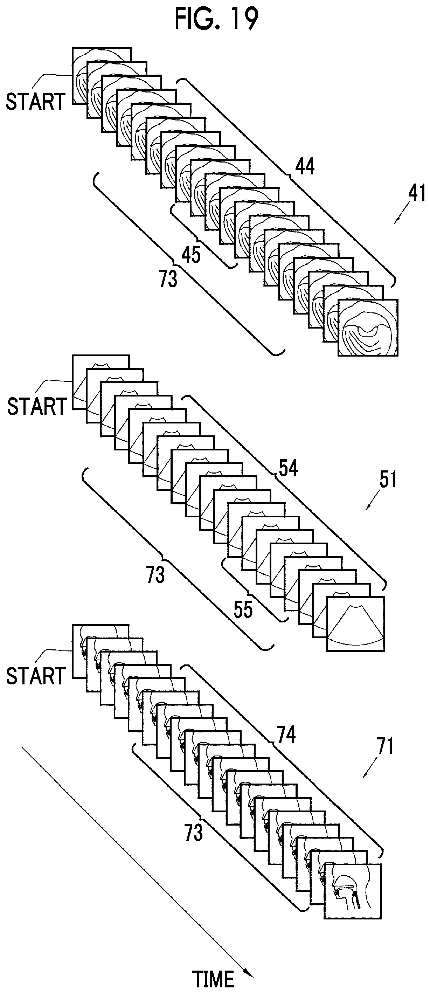

Fig. 19 is an image diagram illustrating the extraction of index moving images from the endoscopic moving image, the ultrasound moving image, and the fluoroscopic moving image.

### DESCRIPTION OF THE PREFERRED EMBODIMENTS

### First Embodiment

As illustrated in Fig. 1, a medical image processing system 10 includes an endoscope system 11, an ultrasound system 12, a processor device 13, a display 14, and a user interface 15. The processor device 13 is electrically connected to the endoscope system 11, the ultrasound system 12, the display 14, and the user interface 15. The processor device 13 acquires video signals acquired by a swallowing examination from an endoscope 11a constituting the endoscope system 11 and an ultrasonography terminal 12a constituting the ultrasound system 12. The user interface 15 is an input device that performs, for example, the input of settings to the processor device 13 and includes a keyboard and the like.

In addition, the endoscope 11a and the ultrasonography terminal 12a are operated by batteries to acquire videos of the swallowing examination of a patient. Since swallowing is a movement, a video signal, which is moving image data, is acquired in the swallowing examination. It is preferable that the video signal is wirelessly transmitted to the processor device 13. In addition, unless otherwise specified in the imaging of swallowing, white light is used as illumination light, a video signal of 60 frame images (60 frames per second (fps)) is acquired per second, and the imaging time based on the processor device 13 is recorded. Further, in a case in which the video signal is 60 fps, it is preferable to count time in centiseconds. It is preferable that the processor device 13, the display 14, and the user interface 15 are integrated into a tablet terminal as a medical image processing system that can be carried together with the endoscope system 11 and the ultrasound system 12.

As illustrated in Fig. 2, swallowing is a series of movements in which food F in the mouth is chewed, swallowed, and transported to the stomach. The progression of the swallowing includes an "oral stage" in which the food F in the mouth is transported from the tongue to the pharynx, a "pharyngeal stage" in which the food F is transported from the pharynx to the esophagus, and an "esophageal stage" in which the food F is transported from the esophagus to the stomach. In this embodiment, the pharynx is examined with the endoscope 11a, and the esophagus is examined with the ultrasonography terminal 12a in the "pharyngeal stage" of the swallowing to mainly acquire a moving image of the "esophageal stage" of the swallowing. A plurality of liquids or solids that are harmless to the human body even in a case in which they are swallowed and have different degrees of easiness to swallow are used as the food F. For example, milk, a colored aqueous solution, and pudding are used as the food F.

In the swallowing examination, an aspect in which the patient puts the food F into the mouth, swallows it in the pharynx, and transports it to the stomach through the esophagus is imaged. Even in a case in which the patient is not able to swallow the food F, imaging is performed without being stopped. In a case in which the swallowing examination is performed, it is preferable not to image the swallowing once but to image the swallowing a plurality of times within a range in which the patient is not stained.

As illustrated in Fig. 3, the endoscope 11a is a swallowing endoscope that is inserted from a nasal cavity Nc of the patient and images the swallowing of the food F around the pharynx illuminated by the illumination light in a state in which an endoscope tip portion 11b, which is a tip, is located near a position R. The ultrasonography terminal 12a is put to the neck of the patient such that an ultrasonography terminal contact portion 12b, which is a tip, comes into contact with the vicinity of a throat Th and images the swallowing of the food F from the airway to the esophagus. In addition, in ultrasonography, it is preferable to apply a special gel in advance to a part of the body surface of the patient with which the ultrasonography terminal contact portion 12b comes into contact. The endoscope tip portion 11b performs imaging substantially horizontally to the ground, and the ultrasonography terminal contact portion 12b performs imaging substantially perpendicularly to the ground. Further, the part with which the ultrasonography terminal 12a comes into contact is changed such that the ultrasonography terminal 12a can image the swallowing in the pharyngeal stage and the oral stage in addition to the esophageal stage.

As illustrated in Fig. 4, in the processor device 13, a program related to processing, such as image processing, is stored in a program memory (not illustrated). In the processor device 13, a central control unit 20 configured by a processor operates the program in the program memory to implement the functions of an input receiving unit 21, a display control unit 22, a storage memory 23, an endoscopic moving image processing unit 24, an ultrasound moving image processing unit 25, and a moving image editing unit 30. Further, with the implement of the functions of the moving image editing unit 30, the functions of a temporary storage memory 31, a moving image synchronization unit 32, a swallowing timing detection unit 33, an index moving image extraction unit 34, and a swallowing classification unit 35 are implemented. In addition, the function of the swallowing timing detection unit 33 may be provided in the endoscope system 11.

An endoscopic video signal acquired by the endoscope system 11 is transmitted to the moving image editing unit 30 through the endoscopic moving image processing unit 24, and an ultrasound video signal acquired by the ultrasound system 12 is transmitted to the moving image editing unit 30 through the ultrasound moving image processing unit 25. The input receiving unit 21 is connected to the user interface 15. The display control unit 22 performs control to display the moving images synchronized by the moving image synchronization unit 32 on the display 14. The storage memory 23 temporarily stores each video signal as a moving image.

In a case in which a moving image is being recorded after the end of the imaging in the swallowing examination, an operation mode is switched to either a normal moving image synchronization mode or an index moving image synchronization mode. The normal moving image synchronization mode stores and synchronizes moving images, and the index moving image synchronization mode stores and synchronizes moving images, extracts index moving images, and classifies the types of swallowing examinations. During the swallowing examination, the operation mode is set to a real-time display mode that displays the captured content in real time.

In a case in which the imaging in the swallowing examination ends, the real-time display mode is switched to either the normal moving image synchronization mode or the index moving image synchronization mode. It is preferable that the user sets which mode to switch to through the user interface 15 before the real-time display mode ends. In addition, the moving image is acquired after the swallowing examination ends. However, each video signal may be processed in real time during the swallowing examination.

The normal moving image synchronization mode will be described. As illustrated in Fig. 5, each acquired video signal is temporarily stored as an endoscopic moving image 41 and an ultrasound moving image 51 in the temporary storage memory 31 and is synchronized by the moving image synchronization unit 32 on the basis of the imaging time. The endoscopic moving image 41 and the ultrasound moving image 51 after the synchronization are arranged on the display 14 through the display control unit 22 and are synchronously played back. Further, a play button 61, a fast rewind button 62, a fast forward button 63, a pause button 64, a seek bar 65, a slider 66, a repeat play button 67, and a screen switching button 68 are provided, and the user can use these functions through the user interface 15.

In the temporary storage memory 31, each video signal, on which the swallowing examination has been recorded and which has been transmitted to the moving image editing unit 30, is temporarily stored as the endoscopic moving image 41 and the ultrasound moving image 51. Each moving image whose temporary storage has been completed is transmitted to the moving image synchronization unit 32.

The moving image synchronization unit 32 performs the synchronization process on the endoscopic moving image 41 and the ultrasound moving image 51 temporarily stored in the temporary storage memory 31. In the synchronization process, the endoscopic moving image 41 and the ultrasound moving image 51 are synchronized in units of frames on the basis of the imaging time such that they can be played back on a playback screen at the same time. In addition, for example, patient information or information, such as the imaging date and time, is also synchronized. Further, the imaging time used as the reference for synchronization is the time when the endoscope system 11 and the ultrasound system 12 are synchronized with the processor device 13 in advance, and the endoscopic moving image 41 and the ultrasound moving image 51 after the synchronization process are transmitted to the display control unit 22.

The endoscopic moving image 41 and the ultrasound moving image 51 subjected to the synchronization process are displayed on the playback screen of the display 14 by the display control unit 22 and are played back automatically or by the operation of the user. The user observes the aspect of simultaneous playback. On the playback screen, the play button 61 can be pressed to repeatedly play back the endoscopic moving image 41 and the ultrasound moving image 51 whose automatic playback has been ended, the fast rewind button 62 can be used to go back to the missed scene, the fast forward button 63 can be used to increase a moving image playback speed, and the pause button 64 can be used to stop playback at any time. The playback state of the moving image is represented by the position of the slider 66 on the seek bar 65, and the position of the slider 66 can be moved to freely change a playback point by the operation of the user such as dragging. In a case in which the repeat play button 67 is selected, the endoscopic moving image 41 and the ultrasound moving image 51 whose playback has been ended are repeatedly played back.

As illustrated in Fig. 6, in the playback of the moving image on the display 14, the screen switching button 68 can be used to switch the display of the endoscopic moving image 41 and the ultrasound moving image 51 synchronized with each other from two-screen synchronized display to one-screen display. It is preferable that the endoscopic moving image 41 is displayed on one screen and the screen switching button 68 is selected again to switch the screen to the one-screen display of the ultrasound moving image 51 and is further selected again to switch the screen to the two-screen synchronized display. In the case of the one-screen display, a moving image information display field 69 may be expanded to display moving image information, such as patient information, such that the user inputs the moving image information through the user interface 15.

The user can check the information of the endoscopic moving image 41 and the ultrasound moving image 51 displayed in the moving image information display field 69 from the user interface 15 through the input receiving unit 21 and perform editing, such as addition and correction, on the information. The information is characteristics, such as the findings obtained by playing back the moving image and the gender or age of the patient. The endoscopic moving image 41 and the ultrasound moving image 51 including the edited moving image information are stored in the storage memory 23.

The index moving image synchronization mode will be described. As in the normal moving image synchronization mode, the endoscopic moving image 41 and the ultrasound moving image 51 are temporarily stored in the temporary storage memory 31, and the moving image synchronization unit 32 performs the synchronization process on the moving images on the basis of the imaging time. For the endoscopic moving image 41 and the ultrasound moving image 51 after the synchronization, the swallowing timing detection unit 33 detects and tags frame images having the swallowing timing. The index moving image extraction unit 34 extracts an index moving image as a scene, in which swallowing has been performed, in order to efficiently observe swallowing. The swallowing classification unit 35 automatically classifies, for example, the types of index moving images. After the automatic classification, the display control unit 22 arranges the index moving images on the display 14 and synchronously plays back the index moving images.

The temporary storage of the moving images in the temporary storage memory 31 and the synchronization process of the moving image synchronization unit 32 are performed in common in both the normal moving image synchronization mode and the index moving image synchronization mode. Therefore, the normal moving image synchronization mode and the index moving image synchronization mode may be switched until the synchronization process is completed.

Fig. 7 illustrates an endoscopic swallowing frame image 42 showing a state in which the food F passes through the pharynx and an endoscopic non-swallowing frame image 43 showing a state in which the food F does not pass through the pharynx, which constitute the endoscopic moving image 41. In the capture of the endoscopic moving image 41, the imaging starts before and after the patient puts the food F in the mouth and ends after swallowing including aspiration is performed. However, since the time required to capture swallowing is several seconds at the longest, the number of endoscopic non-swallowing frame images 43 is large among the frame images constituting the endoscopic moving image 41.

Fig. 8 illustrates an ultrasound swallowing frame image 52 showing a state in which the food F passes through the esophagus and an ultrasound non-swallowing frame image 53 showing a state in which the food F does not pass through the esophagus, which constitute the ultrasound moving image 51. Since the capture of the ultrasound moving image 51 starts and ends in the same manner as the capture of the endoscopic moving image 41, the ultrasound moving image 51 is configured to include a larger number of ultrasound non-swallowing frame images 53 that do not include the food F.

The swallowing timing detection unit 33 performs a pharyngeal stage swallowing detection process that analyzes the endoscopic moving image 41 with a tool only for machine learning using, for example, deep learning to determine whether or not to have a pharyngeal stage swallowing timing which is a movement in a case in which the food F passes through the pharynx. A portion determined to have the pharyngeal stage swallowing timing is the endoscopic swallowing frame image 42 including the food F. In a case in which the swallowing timing is detected by the pharyngeal stage swallowing detection process, the frame image is tagged with "swallowing timing detection".

Further, similar to the endoscopic moving image 41, the swallowing timing detection unit 33 performs an esophageal stage swallowing detection process that analyzes the ultrasound moving image 51 with a tool only for machine learning using, for example, deep learning to determine whether or not to have an esophageal stage swallowing timing which is a movement in a case in which the food F passes through the esophagus. A portion determined to have the esophageal stage swallowing timing is the ultrasound swallowing frame image 52 including the food F. In a case in which the swallowing timing is detected by the esophageal stage swallowing detection process, the frame image is tagged with "swallowing timing detection".

The endoscopic moving image 41 and the ultrasound moving image 51 after the tagging are transmitted to the index moving image extraction unit 34. Further, it is preferable that the endoscopic moving image 41 and the ultrasound moving image 51 from which the swallowing timing has not been detected are stored in the storage memory 23. In this case, the endoscopic moving image 41 and the ultrasound moving image 51 are deleted from the temporary storage memory 31.

The index moving image extraction unit 34 extracts an endoscopic index moving image 44, which is a continuous frame image group including the frame image tagged with the "swallowing timing detection", from the endoscopic moving image 41. Similarly, the index moving image extraction unit 34 extracts an ultrasound index moving image 54, which is a continuous frame image group including the frame image tagged with the "swallowing timing detection", from the ultrasound moving image 51.

Specifically, as illustrated in Fig. 9, the index moving image extraction unit 34 extracts a continuous frame image group from a pharyngeal stage swallowing frame image group 45 to an esophageal stage swallowing frame image group 55, which are tagged with the "swallowing timing detection", in the endoscopic moving image 41 and the ultrasound moving image 51 and frame image groups for a predetermined period which are continuous before and after the continuous frame image group. The predetermined period is a time of about 3 seconds or 5 seconds set in advance by the user and is preferably a period required to capture, for example, the movement of the pharynx before the passage of the food F or the movement of the esophagus after the passage of the food F. An arrow indicates the progress of time. In a case in which the moving image is played back, it is played back and displayed along the direction of the arrow. The endoscopic index moving image 44 and the ultrasound index moving image 54 include the swallowing timing in the pharyngeal stage and the esophageal stage. The extracted endoscopic index moving image 44 and ultrasound index moving image 54 are transmitted to the swallowing classification unit 35.

Since the endoscopic moving image 41 and the ultrasound moving image 51 are synchronized, the index moving images may be extracted such that the tagging result of one of the moving images is synchronized with that of the other moving image. Therefore, for the swallowing timing detected in only one of the moving images, it is possible to check how a video looks in the other moving image.

The swallowing timing captured in the endoscopic moving image 41 is in the pharyngeal stage, and the swallowing timing captured in the ultrasound moving image 51 is in the esophageal stage. Therefore, the periods for which the food F is displayed in a case in which the moving images are played back in synchronization are not matched with each other. Therefore, it is preferable that the extraction range of each index moving image includes both the pharyngeal stage swallowing timing captured in the endoscopic moving image 41 and the esophageal stage swallowing timing captured in the ultrasound moving image 51. In addition, the swallowing in the pharyngeal stage and the esophageal stage is continuous, and the difference between the swallowing timings is less than 1 second. Therefore, it possible to determine that the pharyngeal stage swallowing timing and the esophageal stage swallowing timing detected with a time difference of less than 1 second indicate the same swallowing.

Further, there are a plurality of patterns in the method for performing the swallowing timing detection and the tagging in order to extract each index moving image. A first pattern is a pattern in which the swallowing timing is detected in each of the endoscopic moving image 41 and the ultrasound moving image 51 and tagging is performed. Examples of second to sixth patterns are given below. In a case in which tagging is performed using the synchronization process, the esophageal stage swallowing timing and the pharyngeal stage swallowing timing may be detected as the swallowing timing from each moving image or each image signal, without being distinguished from each other.

In the second pattern, the swallowing timing detected in the endoscopic moving image 41 is tagged, is reflected to a portion having the same imaging time in the synchronized ultrasound moving image 51, and is tagged as the swallowing timing of the ultrasound moving image 51. Therefore, the detection of the swallowing timing in the ultrasound moving image 51 is not performed.

In the third pattern, the swallowing timing detected in the ultrasound moving image 51 is tagged, is reflected to a portion having the same imaging time in the synchronized endoscopic moving image 41, and is tagged as the swallowing timing of the endoscopic moving image 41. Therefore, the detection of the swallowing timing in the endoscopic moving image 41 is not performed.

The swallowing timing may be detected by combining the endoscopic moving image 41 and the ultrasound moving image 51. In the detection of the swallowing timing using a single moving image, the swallowing timing may not be detected or may be erroneously detected. However, the accuracy of detection is improved by the combination of the endoscopic moving image 41 and the ultrasound moving image 51. For example, in a case in which the pharynx moves violently due to coughing or choke, the violent movement is erroneously detected as the swallowing timing since it is indistinguishable from a movement during swallowing in the endoscopic moving image 41. However, in the ultrasound moving image 51, the violent movement can be determined not to be swallowing since the movement of muscle is different and can be excluded from the swallowing timing detected in the endoscopic moving image 41. For example, in the ultrasound moving image 51, an examination using an examination food is not distinguishable from a case in which the patient unconsciously swallows. Therefore, in the ultrasound moving image 51, the unconscious swallowing is detected as the swallowing timing. However, since the inflow and non-inflow of the examination food can be determined in the endoscopic moving image 41, it is possible to exclude swallowing other than the swallowing of the examination food.

In the fourth pattern, after the swallowing timing is detected in the endoscopic moving image 41 and the ultrasound moving image 51, the endoscopic moving image 41 and the ultrasound moving image 51 synchronized with each other are compared, and only the image signals at the swallowing timing having the same imaging time are tagged.

In the fifth pattern, the swallowing timing in the endoscopic moving image 41 and a timing other than the non-swallowing timing in the synchronized ultrasound moving image 51 are tagged. For example, an endoscopic video signal at the swallowing timing is detected from the endoscopic moving image 41, and an ultrasound video signal at least the non-swallowing timing among the swallowing timing, the non-swallowing timing, and an unknown timing is detected from the ultrasound moving image 51. Among the endoscopic video signals at the swallowing timing, an endoscopic video signal having the same imaging time as the ultrasound video signal at the timing other than the non-swallowing timing is tagged. In addition, among the ultrasound video signals at the timing other than the non-swallowing timing, an ultrasound video signal having the same imaging time as the endoscopic video signal at the swallowing timing is tagged.

In the sixth pattern, the swallowing timing in the ultrasound moving image 51 and a timing other than the non-swallowing timing in the synchronized endoscopic moving image 41 are tagged. For example, an ultrasound video signal at the swallowing timing is detected from the ultrasound moving image 51, and an endoscopic video signal at least the non-swallowing timing among the swallowing timing, the non-swallowing timing, and the unknown timing is detected from the endoscopic moving image 41. Among the ultrasound video signals at the swallowing timing, an ultrasound video signal having the same imaging time as the endoscopic video signal at the timing other than the non-swallowing timing is tagged. In addition, among the endoscopic video signals at the timing other than the non-swallowing timing, an endoscopic video signal having the same imaging time as the ultrasound video signal at the swallowing timing is tagged.

The swallowing classification unit 35 classifies the types of swallowing foods in the swallowing examination or the results of the swallowing examinations obtained by analyzing the endoscopic index moving image 44 and the ultrasound index moving image 54 or gives, for example, index numbers. The swallowing food is the type of the food F swallowed and is, for example, saliva, milk, a colored aqueous solution, and pudding. In addition, the results of normal swallowing, aspiration, and unknown swallowing are classified. Score evaluation that gives a point according to the degree of aspiration may be used. These are automatically classified on the basis of data learned by machine learning. It is preferable that information on the automatically classified types of swallowing examination foods is given to the endoscopic index moving image 44 and the ultrasound index moving image 54. The endoscopic index moving image 44 and the ultrasound index moving image 54 after the classification are transmitted to the display control unit 22. In addition, the result of the swallowing examination may be determined by the swallowing timing detection unit 33, and the result of the determination may be used.

The index numbers are numbers that are used by the user to search for each index moving image stored in, for example, the storage memory 23 and are preferably alphanumeric character strings that do not overlap each other, have a small number of digits, and indicate the order in which the swallowing timing is detected and the type of swallowing examination food. In this case, the types of swallowing examination foods are classified into saliva (sa), milk (mi), colored water (cw), pudding (pu), and unknown (un). In addition, the results of swallowing are classified into normal swallowing (S), aspiration (A), and unknown swallowing (U). Further, (1) is added to the end of the endoscopic index moving image 44, and (2) is added to the end of the ultrasound index moving image 54, depending on the type of moving image.

As illustrated in Fig. 10, for example, the swallowing classification unit 35 specifies the type of swallowing in endoscopic index moving images 44a, 44b, 44c, 44d, and 44e extracted continuously and ultrasound index moving images 54a, 54b, 54c, 54d, and 54e synchronized with these endoscopic index moving images. One swallowing examination result is obtained from the endoscopic index moving image and the ultrasound index moving image synchronized with each other. Symbols indicating the swallowing examination food, the result of the swallowing examination, and the type of moving image are incorporated in the index number indicating the order in which the swallowing timing is detected. Assuming that the endoscopic index moving images 44a to 44e are sequentially extracted in the order in which the swallowing timing is detected, 44a shows the normal swallowing of the saliva and is represented by "001saS1", 44b shows the normal swallowing of the milk and is represented by "002miS1", 44c shows the unknown swallowing of the colored water and is represented by "003cwU1", 44d shows the aspiration of the pudding and is represented by "004puA1", and 44e shows the normal swallowing of the unknown swallowing examination food and is represented by "005unS1". In a case in which the ultrasound index moving images 54a to 54e synchronized with the endoscopic index moving images 44a to 44e have the same result, 54a represented by "001saS2", 54b represented by "002miS2", 54c represented by "003cwU2", 54d represented by "004puA2", and 54e represented by "005unS2".

For example, the display control unit 22 controls the switching of the screen displayed on the display 14. During the swallowing examination, a real-time video observed by the user is displayed as an observation screen. In a case in which imaging ends and an acquired moving image or the like is displayed, the moving image is displayed as a playback screen. The playback of the moving image on the observation screen is performed automatically or by the operation of the user.

The moving image editing unit 30 automatically performs processes from the acquisition of the endoscopic moving image 41 and the ultrasound moving image 51 to the display of the endoscopic index moving image 44 and the ultrasound index moving image 54 on the screen. In a case in which the imaging by the endoscope 11a and the ultrasonography terminal 12a ends, the display of the display 14 is switched from the observation screen to the playback screen. The endoscopic index moving image 44 extracted from the acquired endoscopic moving image 41 and the ultrasound index moving image 54 extracted from the acquired ultrasound moving image 51 are displayed on the playback screen.

In a case in which tagging or information addition is performed on one of the endoscopic index moving image 44 and the ultrasound index moving image 54 synchronized with each other, tagging or information addition is performed on the other moving image in synchronization. For example, in a case in which any frame image of the endoscopic index moving image 44 is tagged with "swallowing timing", the corresponding frame image of the ultrasound moving image 51 synchronized by the synchronization process is also tagged with "swallowing timing".

Synchronization content is synchronized with each corresponding frame image in centiseconds on the basis of the imaging time. In addition to the content of the tagging, the classification result of the swallowing classification unit 35 or the editing result of the moving image editing unit 30 is also reflected. Further, any content designated by the endoscopic video signal and the ultrasound video signal may not be synchronized.

As illustrated in Fig. 11, two types of moving images, for example, the endoscopic index moving image 44 and the ultrasound index moving image 54 on the playback screen displayed on the display 14 are simultaneously displayed and are synchronously played back as in Fig. 5. Therefore, the pharynx and the esophagus can be observed at the same time, and the movement of the food F in the esophagus from the pharynx can be continuously observed at the same time. The playback screen comprises the play button 61, the fast rewind button 62, the fast forward button 63, the pause button 64, the seek bar 65, the slider 66, the repeat play button 67, and the screen switching button 68 for the user to operate the playback of the moving image, and the user can use these functions through the user interface 15.

As illustrated in Fig. 12, the endoscopic index moving image 44 and the ultrasound index moving image 54 on the playback screen displayed on the display 14 may be switched from the simultaneous playback illustrated in Fig. 11 to be independently displayed by the screen switching button 68 as in Fig. 6. In the case of the independent display, the moving image information display field 69 may be expanded to further display the type of swallowing and the index number given by the swallowing classification unit 35. Further, the index number may be applied as the moving image title of the endoscopic index moving image 44.

The user can check the information of the endoscopic index moving image 44 and the ultrasound index moving image 54 displayed in the moving image information display field 69 from the user interface 15 through the input receiving unit 21 and perform editing, such as addition and correction, on the information. The information is characteristics, such as the findings obtained by playing back the moving image and the gender or age of the patient. In addition, the automatically classified types of swallowing examination foods or the presence or absence of aspiration may be edited.

The user can edit the extraction range of the index moving image in addition to editing the information in the moving image information display field 69. For example, in a case in which the result of the review of the playback screen shows that the swallowing timing is erroneously detected or the extraction is insufficient, the endoscopic index moving image 44 and the ultrasound index moving image 54 having the swallowing timing may be reacquired by re-extraction including manual extraction or re-examination with reference to the endoscopic moving images 41 stored in the temporary storage memory 31.

The endoscopic index moving image 44 and the ultrasound index moving image 54 in which various kinds of moving image information have been checked and edited are stored in the storage memory 23. In this case, it is preferable to delete the endoscopic moving image 41 and the ultrasound moving image 51 which are the extraction sources stored in the temporary storage memory 31.

In deep learning in the swallowing detection process for the endoscopic moving image 41 and the ultrasound moving image 51, a tool learns the characteristics of an image, which is the object to be detected, in advance. Then, the tool calculates the probability that the analyzed frame image will be the object to be detected, and the frame image having a probability equal to or greater than a threshold value is determined to be the object to be detected. The object to be detected is the pharynx that captures the food F, and the movement of the food F is tracked. The endoscopic swallowing frame image 42 or the ultrasound swallowing frame image 52 is detected and tagged. Further, in deep learning, it is necessary to learn information corresponding to the type of food F used for the swallowing examination.

The pharyngeal stage swallowing timing recorded in the endoscopic moving image 41 is the movement of the pharynx in a case in which the patient swallows the food F. However, deep learning for recognizing the food F is not needed to detect the pharyngeal stage swallowing timing, and the swallowing detection process may be performed according to, for example, the characteristics of the image during swallowing or a change between the front and rear frame images. Specifically, the swallowing detection process may be performed by a swallowing detection algorithm using, for example, "a difference between the pixel values of the front and rear frame images", "the amount of blur of the frame image", and "the number of key points of the image". In addition, in any swallowing detection process, the frame image determined to shows the swallowing state is tagged with "swallowing timing detection".

In the detection algorithm using "the difference between the pixel values of the front and rear frame images", since the amount of movement of the object between the frame images is large in the images showing the swallowing state, the difference between the simple pixel values of the front and rear frame images is calculated. The swallowing timing detection unit 33 calculates the difference between the pixel values of two frame images that are continuous in time series. It is preferable to use AbsDiff installed in OpenCV (registered trademark) used for image analysis as a function for calculating the difference (simple difference value).

In the detection algorithm using "the amount of blur of the frame image", since the amount of blur of the object between the frame images is large in the images showing the swallowing state, the amount of edge indicating the amount of blur between the frame images is calculated. The swallowing timing detection unit 33 calculates the amount of edge related to the two frame images that are continuous in time series. It is preferable to use Variance Of Laplacian installed in OpenCV (registered trademark) used for image analysis as a function for calculating the amount of edge.

In the detection algorithm using the "number of key points", since the images showing the swallowing state have a large amount of blur of the object between the frame images, the edges of the frame images are unclear and the number of key points is reduced. The key point means a feature point which is a portion having a high probability of being a corner with a large amount of edge among the edges obtained by extracting lines representing the frame image. The swallowing timing detection unit 33 calculates the number of key points related to the frame image. It is preferable to use Count Key Points installed in OpenCV (registered trademark) used for image analysis as a function for calculating the number of key points. In addition, Accelerated KAZE (AKAZE) installed in OpenCV (registered trademark) used for image analysis is used to extract the feature points. In the extraction of the feature points in this embodiment, it is preferable to recognize a portion (a portion recognized as a "corner") having a large amount of edge in the image.

The real-time display mode will be described. The real-time videos that have not been subjected to the synchronization process during imaging by the endoscope system 11 and the ultrasound system 12 are displayed side by side on the display 14. A video signal captured by starting pharynx endoscopy with the endoscope system 11 is transmitted to the display control unit 22 through the endoscopic moving image processing unit 24 of the processor device 13. Further, a video signal captured by starting esophagus ultrasonography with the ultrasound system 12 is transmitted to the display control unit 22 through the ultrasound moving image processing unit 25 of the processor device 13. The display control unit 22 displays the video signals as real-time videos on the display 14. In addition, in a case in which the captured moving image of the swallowing examination is recorded, it is preferable that the user decides the start and end of the recording with a switch or the like provided in the endoscope 11a and the ultrasonography terminal 12a.

Fig. 13 is a flowchart illustrating a series of flow of acquiring index moving images of the swallowing examination with two systems using endoscopic moving image capture and ultrasound moving image capture. The flow of extracting and playing back the endoscopic index moving image 44 and the ultrasound index moving image 54 in the swallowing examination will be described below. The user acquires the endoscopic moving image 41 obtained by capturing the aspect of the swallowing examination in the pharynx with the endoscope 11a and the ultrasound moving image 51 obtained by capturing the aspect of the swallowing examination in the esophagus with the ultrasonography terminal 12a in the real-time display mode. After the imaging of the swallowing examination ends, the operation mode is switched to the normal moving image synchronization mode or the index moving image synchronization mode. In any case, the acquired endoscopic moving image 41 is transmitted to the moving image editing unit 30 through the endoscopic moving image processing unit 24, and the acquired ultrasound moving image 51 is transmitted to the moving image editing unit 30 through the ultrasound moving image processing unit 25. The endoscopic moving image 41 and the ultrasound moving image 51 transmitted to the moving image editing unit 30 are stored in the temporary storage memory 31 and then synchronized by the moving image synchronization unit 32.

In the normal moving image synchronization mode, the endoscopic moving image 41 and the ultrasound moving image 51 after the synchronization process are displayed on the display 14 and are synchronously played back such that the swallowing timing is observed from two types of images. In the index moving image synchronization mode, the swallowing timing detection unit 33 analyzes the endoscopic moving image 41 and the ultrasound moving image 51 synchronized with each other to specify the pharyngeal stage swallowing frame image group 45 or the esophageal stage swallowing frame image group 55 which are frame images capturing the swallowing timing. From the endoscopic moving image 41 and the ultrasound moving image 51 transmitted to the index moving image extraction unit 34 next to the swallowing timing detection unit 33, the frame images from the pharyngeal stage swallowing frame image group 45 to the esophageal stage swallowing frame image group 55 and the frame images for a predetermined period of time before and after the frame image groups are extracted as the endoscopic index moving image 44 and the ultrasound index moving image 54. The extracted endoscopic index moving image 44 and ultrasound index moving image 54 are transmitted to the swallowing classification unit 35, and the classification of the type of swallowing examination and the giving of the index numbers are performed by machine learning. Then, the endoscopic index moving image 44 and the ultrasound index moving image 54 are transmitted to the display control unit 22, are displayed on the display 14, and are synchronously played back such that the swallowing timing is observed from the two types of images in a short time.

After the moving images synchronously played back in the normal moving image synchronization mode and the index moving image synchronization mode are observed, it is possible to check, for example, the type of swallowing examination and to perform editing such as addition and correction. Each moving image whose moving image information has been edited is stored in the storage memory 23 in the processor device 13. In this case, it is preferable to delete the endoscopic moving image 41 and the ultrasound moving image 51 stored in the temporary storage memory 31 in a case in which they are not needed. In a case in which the number of moving images acquired is insufficient due to, for example, an extraction failure or in a case in which image quality is poor, re-examination is performed to acquire moving images again.

Ultrasonography may evaluate aspiration and residue in the laryngopharynx and evaluate the movement of muscles related to swallowing, in addition to the above-mentioned observation of swallowing in the esophageal stage. The target part is observed with the ultrasonography terminal 12a to acquire an ultrasound video signal. It is preferable to analyze the ultrasound video signal to detect the swallowing timing and to extract the index moving image. **In** the observation of swallowing by ultrasonography, the swallowing detection process is mainly an esophageal stage swallowing detection process. However, in non-invasive ultrasonography, an observation part can be easily changed. Therefore, an oral stage swallowing detection process or a pharyngeal stage swallowing detection process is also performed for the food F located at the position illustrated in Fig. 2. In addition, since the depth from the body surface or the shape of the body surface differs depending on each observation part, a plurality of ultrasonography terminals 12a may be used properly according to the observation content of the ultrasonography.

In the evaluation of aspiration and residue in the laryngopharynx, the same laryngopharynx as that in the observation with the swallowing endoscope is set as the observation part, and the aspiration and residue of food and beverage in the pharyngeal stage are evaluated. The ultrasound moving image 51 of the laryngopharynx is acquired by the ultrasonography terminal 12a, and the images at the time of swallowing are learned by deep learning to detect the swallowing timing. The ultrasound index moving image 54 can be extracted by synchronizing the detected swallowing timing with the endoscopic moving image 41. The types and results of swallowing foods by endoscopy and the evaluation of aspiration and residual by ultrasonography may be displayed on the screen so as to be associated with each other.

In the evaluation of the movement of the muscles related to swallowing, the morphology of the tongue muscles, muscles around the hyoid bone, or the larynx muscles is observed to evaluate the swallowing movement. The observation of the tongue or the vicinity of the hyoid bone is the examination of the swallowing timing in the oral stage, and the observation of the muscles of the larynx is the examination of the swallowing timing in the pharyngeal stage. The ultrasound moving image 51 is acquired by the ultrasonography terminal 12a, and the images at the time of swallowing in the oral stage or the pharyngeal stage are learned by deep learning to detect the swallowing timing. The ultrasound index moving image 54 can be extracted by synchronizing the detected swallowing timing with the endoscopic moving image 41. The types and results of swallowing foods by endoscopy and the evaluation of the movement of the muscles related to swallowing by ultrasonography may be displayed on the screen so as to be associated with each other.

### Second Embodiment

In this embodiment, instead of the ultrasound system 12 according to the first embodiment, as illustrated in Fig. 14, a radiation observation system 16 having a radiation observation device 16a is used to acquire a video signal, and the swallowing examination of the patient is observed. The endoscopic moving image 41 and a fluoroscopic moving image 71 according to this embodiment will be described below. In addition, the description of the same content as that in the above-described embodiment will not be repeated.

The radiation observation system 16 is composed of the radiation observation device 16a. The radiation observation device 16a images that the patient swallows the food F containing a contrast medium and acquires the fluoroscopic moving image 71. Further, in the processor device 13, the function of a fluoroscopic moving image processing unit (not illustrated) that receives the fluoroscopic moving image 71 captured by the radiation observation system 16 and transmits the fluoroscopic moving image 71 to the moving image editing unit 30 is implemented.

As illustrated in Fig. 15, the endoscopic moving image 41 and the fluoroscopic moving image 71 synchronized by the moving image synchronization unit 32 are transmitted to the display control unit 22 and are simultaneously displayed and synchronously played back on the display 14. The fluoroscopic moving image 71 can include a series of continuous swallowing movements including the oral stage in which the food F is transported from the oral cavity to the pharynx by the tongue, the pharyngeal stage in which the food F is transported from the pharynx to the esophagus, and the esophageal stage in which the food F is transported from the esophagus to the stomach as illustrated in Fig. 2. Therefore, it is preferable to image the patient from the side and to displays a video including the oral cavity, the pharynx, and the esophagus on the display 14.

As illustrated in Fig. 16, a group 73 of a series of swallowing frame images capturing the oral stage to the esophageal stage included in the fluoroscopic moving image 71 includes the entire pharyngeal stage swallowing frame image group 45 that captures swallowing in the pharyngeal stage. Therefore, for the endoscopic index moving image 44 and a radiation index moving image 74, the group 73 of a series of swallowing frame images and a frame image group for a predetermined period before and after the group 73 of a series of swallowing frame images are extracted from the endoscopic moving image 41 and the fluoroscopic moving image 71.

In the swallowing examination using the radiation observation system 16, in the swallowing classification unit 35, the type of food F is different from that in the first embodiment since a contrast medium is used. Therefore, the classification of the type of swallowing is different from that in the first embodiment. For the index number, (1) is added to the end of the endoscopic index moving image 44, and (3) is added to the end of the radiation index moving image 74, depending on the type of moving image. The endoscopic index moving image 44 and the radiation index moving image 74 after the classification are transmitted to the display control unit 22, are displayed on the display 14, and are synchronously played back.

### Third Embodiment

In the above-described embodiment, the endoscope system 11 is fused with a different imaging system, and the swallowing examination is recorded by two systems. However, in this embodiment, three types of imaging systems are fused to observe the swallowing examination.

As illustrated in Fig. 17, in a medical image processing system 10, the swallowing examination is observed by fusing three systems using the endoscope system 11, the ultrasound system 12, and the radiation observation system 16. The review of the swallowing examination according to this embodiment will be described below. In addition, the description of the same content as that in the above-described embodiment will not be repeated.

As illustrated in Fig. 18, the endoscopic moving image 41, the ultrasound moving image 51, and the fluoroscopic moving image 71 synchronized by the moving image synchronization unit 32 are transmitted to the display control unit 22, are simultaneous displayed, and synchronously played back on the display 14. The screen switching button 68 may be used to switch the display from the three-type simultaneous display to the individual display illustrated in Fig. 6.

As illustrated in Fig. 19, the group 73 of a series of swallowing frame images capturing the oral stage to the esophageal stage included in the fluoroscopic moving image 71 includes all of the pharyngeal stage swallowing frame image group 45 that captures swallowing in the pharyngeal stage and the esophageal stage swallowing frame image group 55 that captures swallowing in the esophageal stage. Therefore, for the endoscopic index moving image 44, the ultrasound index moving image 54, and the radiation index moving image 74, the group 73 of a series of swallowing frame images and a frame image group for a predetermined period before and after the group 73 of a series of swallowing frame images are extracted from the endoscopic moving image 41, the ultrasound moving image 51, and the fluoroscopic moving image 71.

In each of the above-described embodiments, for example, the following various processors can be used as a hardware structure of processing units performing various processes, such as the display control unit 22, the storage memory 23, the endoscopic moving image processing unit 24, the ultrasound moving image processing unit 25, the moving image editing unit 30, the temporary storage memory 31, the moving image synchronization unit 32, the swallowing timing detection unit 33, the index moving image extraction unit 34, and the swallowing classification unit 35. The various processors include, for example, a central processing unit (CPU) which is a general-purpose processor that executes software (programs) to function as various processing units, a programmable logic device (PLD), such as a field programmable gate array (FPGA), that is a processor whose circuit configuration can be changed after manufacture, and a dedicated electric circuit that is a processor having a dedicated circuit configuration designed to perform various processes.

One processing unit may be configured by one of the various processors or a combination of two or more processors of the same type or different types (for example, a combination of a plurality of FPGAs and a combination of a CPU and an FPGA). Further, a plurality of processing units may be configured by one processor. A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system-on-chip (SoC). As described above, various processing units are configured using one or more of the various processors as a hardware structure.

In addition, specifically, the hardware structure of the various processors is an electric circuit (circuitry) obtained by combining circuit elements such as semiconductor elements. Further, the hardware structure of the storage unit is a storage device such as a hard disc drive (HDD) or a solid state drive (SSD).

### Explanation of References

10: medical image processing system
11: endoscope system
11a: endoscope
11b: endoscope tip portion
12: ultrasound system
12a: ultrasonography terminal
12b: ultrasonography terminal contact portion
13: processor device
14: display
15: user interface
16: radiation observation system
16a: radiation observation device
20: central control unit
21: input receiving unit
22: display control unit
23: storage memory
24: endoscopic moving image processing unit
25: ultrasound moving image processing unit
30: moving image editing unit
31: temporary storage memory
32: moving image synchronization unit
33: swallowing timing detection unit
34: index moving image extraction unit
35: swallowing classification unit
41: endoscopic moving image
42: endoscopic swallowing frame image
43: endoscopic non-swallowing frame image
44: endoscopic index moving image
44a: endoscopic index moving image
44b: endoscopic index moving image
44c: endoscopic index moving image
44d: endoscopic index moving image
44e: endoscopic index moving image
45: pharyngeal stage swallowing frame image group
51: ultrasound moving image
52: ultrasound swallowing frame image
53: ultrasound non-swallowing frame image
54: ultrasound index moving image
54a: ultrasound index moving image
54b: ultrasound index moving image
54c: ultrasound index moving image
54d: ultrasound index moving image
54e: ultrasound index moving image
55: esophageal stage swallowing frame image group
60: moving image information display field
61: play button
62: fast rewind button
63: fast forward button
64: pause button
65: slider
66: seek bar
67: repeat play button
68: screen switching button
71: fluoroscopic moving image
73: group of series of swallowing frame images
74: radiation index moving image
F: food
Th: throat
Nc: nasal cavity
R: position

## Claims

1. A medical image processing system comprising:
a processor (13) configured to:
receive an endoscopic video signal obtained by imaging a swallowing examination of a throat of a patient with an endoscope (11a) that is inserted from a nasal cavity of the patient;
receive an ultrasound video signal of an esophagus, an airway, a laryngopharynx, a tongue, or a vicinity of a hyoid bone acquired by an ultrasonography terminal (12a) that comes into contact with a neck of the patient;
record an imaging time based on a time of the processor in a case in which the endoscopic video signal and the ultrasound video signal are acquired;
and
simultaneously display the endoscopic video signal and the ultrasound video signal on a display,wherein, during imaging in the swallowing examination, an operation mode is set to a real-time display mode that displays the endoscopic video signal and the ultrasound video signal in real time,
wherein, after the end of the imaging in the swallowing examination, the operation mode is switched to an index moving image synchronization mode, and
wherein, in the index moving image synchronization mode, the processor (13) is further configured to:
perform a synchronization process for the endoscopic video signal and the ultrasound video signal on the basis of the imaging time, the endoscopic video signal and the ultrasound video signal including a frame image group in which frame images are continuous;
detect a swallowing timing from the endoscopic video signal;
detect a swallowing timing from the ultrasound video signal;
tag an endoscopic swallowing frame image (42) and an ultrasound swallowing frame image (52) synchronized according to the swallowing timing from the endoscopic video signal and the swallowing timing from the ultrasound video signal;
extract an endoscopic index moving image which is a continuous frame image group including the endoscopic swallowing frame image identified with the swallowing timing detection;
extract an ultrasound index moving image which is a continuous frame image group including the ultrasound swallowing frame image identified with the swallowing timing detection; and
play back the endoscopic index moving image and the ultrasound index moving image on the display.

2. The medical image processing system according to claim 1,
wherein the processor is configured to:
synchronously play back the endoscopic video signal and the ultrasound video signal subjected to the synchronization process on the display.

3. The medical image processing system according to claim 2,
wherein the endoscopic video signal and the ultrasound video signal are played back automatically or by an operation of a user.

4. The medical image processing system according to claim 1,
wherein the processor is configured to:
detect a non-swallowing timing from the ultrasound video signal;
identify the endoscopic video signal having the same imaging time as the ultrasound video signal at a timing other than the non-swallowing timing among the endoscopic video signals at the swallowing timing and extract an endoscopic index moving image;
identify the ultrasound video signal having the same imaging time as the endoscopic video signal at the swallowing timing among the ultrasound video signals at the timing other than the non-swallowing timing and extract an ultrasound index moving image.

5. The medical image processing system according to claim 4,
wherein the endoscopic index moving image (44) and the ultrasound index moving image (54) are played back automatically or by an operation of a user.

6. The medical image processing system according to any one of claims 4 or 5,
wherein the processor is configured to detect any one of normal swallowing, aspiration, or unknown swallowing as the swallowing timing from the endoscopic video signal and the ultrasound video signal.

7. The medical image processing system according to any one of claims 4 to 6,
wherein the processor is configured to determine whether or not the swallowing timing is present in the endoscopic video signal, using any one of calculation of an amount of blur between the frame images, calculation of the number of key points based on the frame image, or a difference between pixel values of the frame images and determine whether or not the swallowing timing is present in the ultrasound video signal using machine learning.

8. The medical image processing system according to any one of claims 4 to 7,
wherein the processor is configured to analyze at least one of the endoscopic index moving image or the ultrasound index moving image to specify a type of the swallowing examination.

9. The medical image processing system according to claim 8,
wherein the processor is configured to classify the types of the swallowing examinations according to a type of swallowing examination food and a result of swallowing and give index numbers to the endoscopic index moving image and the ultrasound index moving image on the basis of the classification.

10. The medical image processing system according to any one of claims 1 to 9,
wherein the processor is configured to perform imaging at a frame rate of 60 fps in the acquisition of the endoscopic video signal and the ultrasound video signal, and
time is counted in centiseconds in the imaging.

## Patentansprüche

1. Verarbeitungssystem für medizinische Bilder, umfassend:
einen Prozessor (13), der so konfiguriert ist, dass er:
ein endoskopisches Videosignal empfängt, das durch Abbilden einer Schluckuntersuchung eines Rachens eines Patienten mit einem Endoskop (11a), das von einer Nasenhöhle des Patienten eingeführt wird, erhalten wird;
ein Ultraschallvideosignal von einer Speiseröhre, einem Atemweg, einem Laryngopharynx, einer Zunge oder einer Nähe eines Zungenbeins, das von einem Ultraschallterminal (12a), das mit einem Hals des Patienten in Kontakt kommt, erfasst wird, empfängt;
eine Bildgebungszeit auf der Grundlage einer Zeit des Prozessors in einem Fall, in dem das endoskopische Videosignal und das Ultraschallvideosignal erfasst werden, aufzeichnet;
und
das endoskopische Videosignal und das Ultraschallvideosignal gleichzeitig auf einer Anzeige anzeigt, wobei während Bildgebung bei der Schluckuntersuchung ein Betriebsmodus auf einen Echtzeit-Anzeigemodus eingestellt wird, der das endoskopische Videosignal und das Ultraschallvideosignal in Echtzeit anzeigt,
wobei nach dem Ende der Bildgebung bei der Schluckuntersuchung der Betriebsmodus auf einen Index-Bewegtbild-Synchronisationsmodus umgeschaltet wird, und
wobei der Prozessor (13) bei dem Index-Bewegtbild-Synchronisationsmodus ferner so konfiguriert ist, dass er:
einen Synchronisationsprozess für das endoskopische Videosignal und das Ultraschallvideosignal auf der Grundlage der Bildgebungszeit durchführt, wobei das endoskopische Videosignal und das Ultraschallvideosignal eine Einzelbildgruppe, in der Einzelbilder kontinuierlich sind, enthalten;
einen Schluckzeitpunkt aus dem endoskopischen Videosignal detektiert;
einen Schluckzeitpunkt aus dem Ultraschallvideosignal detektiert;
ein endoskopisches Schluckeinzelbild (42) und ein Ultraschall-Schluckeinzelbild (52), die gemäß dem Schluckzeitpunkt aus dem endoskopischen Videosignal und dem Schluckzeitpunkt aus dem Ultraschallvideosignal synchronisiert sind, markiert;
ein endoskopisches Index-Bewegtbild extrahiert, das eine kontinuierliche Einzelbildgruppe ist, die das mit der Schluckzeitpunkt-Detektion identifizierte endoskopische Schluckeinzelbild enthält;
ein Ultraschall-Index-Bewegtbild extrahiert, das eine kontinuierliche Einzelbildgruppe ist, die das mit der Schluckzeitpunkt-Detektion identifizierte Ultraschall-Schluckeinzelbild enthält; und
das endoskopische Index-Bewegtbild und das Ultraschall-Index-Bewegtbild auf der Anzeige wiedergibt.

2. Verarbeitungssystem für medizinische Bilder nach Anspruch 1,
wobei der Prozessor so konfiguriert ist, dass er:
das endoskopische Videosignal und das Ultraschallvideosignal, das dem Synchronisationsprozess unterzogen wurde, auf dem Display synchron wiedergibt.

3. Verarbeitungssystem für medizinische Bilder nach Anspruch 2,
wobei das endoskopische Videosignal und das Ultraschallvideosignal automatisch oder durch eine Bedienung seitens eines Benutzers wiedergegeben werden.

4. Verarbeitungssystem für medizinische Bilder nach Anspruch 1,
wobei der Prozessor so konfiguriert ist, dass er:
einen Nicht-Schluckzeitpunkt aus dem Ultraschallvideosignal detektiert;
das endoskopische Videosignal mit der gleichen Bildgebungszeit als das Ultraschallvideosignal zu einem Zeitpunkt, der von dem Nicht-Schluckzeitpunkt verschieden ist, unter den endoskopischen Videosignalen zu dem Schluckzeitpunkt identifiziert und ein endoskopisches Index-Bewegtbild extrahiert;
das Ultraschallvideosignal mit der gleiche Bildgebungszeit als das endoskopische Videosignal zu dem Schluckzeitpunkt unter den Ultraschallvideosignalen zu dem Zeitpunkt, der von dem Nicht-Schluckzeitpunkt verschieden ist, identifiziert und ein Ultraschall-Index-Bewegtbild extrahiert.

5. Verarbeitungssystem für medizinische Bilder nach Anspruch 4,
wobei das endoskopische Index-Bewegtbild (44) und das Ultraschall-Index-Bewegtbild (54) automatisch oder durch eine Bedienung seitens eines Benutzers wiedergegeben werden.

6. Verarbeitungsvorrichtung für medizinische Bilder nach Anspruch 4 oder 5,
wobei der Prozessor so konfiguriert ist, dass er aus dem endoskopischen Videosignal und dem Ultraschallvideosignal eines von normalem Schlucken, Aspiration und unbekanntem Schlucken als den Schluckzeitpunkt detektiert.

7. Verarbeitungsvorrichtung für medizinische Bilder nach einem der Ansprüche 4 bis 6,
wobei der Prozessor so konfiguriert ist, dass er unter Verwendung einer von Berechnung eines Betrags an Unschärfe zwischen den Einzelbildbildern, Berechnung der Anzahl an Schlüsselpunkten auf der Grundlage des Einzelbildbildes oder einer Differenz zwischen Pixelwerten der Einzelbildbilder bestimmt, ob der Schluckzeitpunkt in dem endoskopischen Videosignal vorhanden ist oder nicht, und unter Verwendung von maschinellem Lernen bestimmt, ob der Schluckzeitpunkt in dem Ultraschallvideosignal vorhanden ist oder nicht.

8. Verarbeitungsvorrichtung für medizinische Bilder nach einem der Ansprüche 4 bis 7,
wobei der Prozessor so konfiguriert ist, dass er mindestens eines von dem endoskopischen Index-Bewegtbild und dem Ultraschall-Index-Bewegtbild analysiert, um einen Typ der Schluckuntersuchung zu spezifizieren.

9. Verarbeitungssystem für medizinische Bilder nach Anspruch 8,
wobei der Prozessor so konfiguriert ist, dass er die Typen der Schluckuntersuchungen gemäß einem Typ von Schluckuntersuchungsnahrung und einem Ergebnis von Schlucken klassifiziert und den endoskopischen Index-Bewegtbild und dem Ultraschall-Index-Bewegtbild auf der Grundlage der Klassifizierung Indexnummern gibt.

10. Verarbeitungsvorrichtung für medizinische Bilder nach einem der Ansprüche 1 bis 9,
wobei der Prozessor so konfiguriert ist, dass er Bildgebung mit einer Bildrate von 60 fps bei der Erfassung des endoskopischen Videosignals und des Ultraschallvideosignals durchführt, und
Zeit bei der Bildgebung in Hundertstelsekunden gezählt wird.

## Revendications

1. Système de traitement d'images médicales comprenant :
un processeur (13) configuré pour :
recevoir un signal vidéo endoscopique obtenu en imaginant un examen de déglutition d'une gorge d'un patient avec un endoscope (11a) qui est inséré à partir d'une cavité nasale du patient ;
recevoir un signal vidéo ultrasonore d'un œsophage, d'une voie respiratoire, d'un laryngopharynx, d'une langue ou d'un voisinage d'un os hyoïde acquis par un terminal d'ultrasonographie (12a) qui entre en contact avec un cou du patient ;
enregistrer un temps d'imagerie sur la base d'un temps du processeur dans un cas où le signal vidéo endoscopique et le signal vidéo ultrasonore sont acquis ;
et
afficher simultanément le signal vidéo endoscopique et le signal vidéo ultrasonore sur un affichage, dans lequel, pendant l'imagerie de l'examen de déglutition, un mode d'opération est réglé sur un mode d'affichage en temps réel qui affiche le signal vidéo endoscopique et le signal vidéo ultrasonore en temps réel,
dans lequel, après la fin de l'imagerie de l'examen de déglutition, le mode d'opération est commuté en un mode de synchronisation d'images en mouvement indexées, et
dans lequel, dans le mode de synchronisation d'images en mouvement indexées, le processeur (13) est en outre configuré pour :
effectuer un processus de synchronisation pour le signal vidéo endoscopique et le signal vidéo ultrasonore sur la base du temps d'imagerie, le signal vidéo endoscopique et le signal vidéo ultrasonore incluant un groupe d'images de trame dans lequel des images de trame sont continues ;
détecter un instant de déglutition à partir du signal vidéo endoscopique ;
détecter un instant de déglutition à partir du signal vidéo ultrasonore ;
étiqueter une image de trame de déglutition endoscopique (42) et une image de trame de déglutition ultrasonore (52) synchronisées en fonction de l'instant de déglutition à partir du signal vidéo endoscopique et de l'instant de déglutition à partir du signal vidéo ultrasonore ;
extraire une image en mouvement indexée endoscopique qui est un groupe d'images de trame continues incluant l'image de trame de déglutition endoscopique identifiée par la détection de l'instant de déglutition ;
extraire une image en mouvement indexée ultrasonore qui est un groupe d'images de trame continues incluant l'image de trame de déglutition ultrasonore identifiée par la détection de l'instant de déglutition ; et
lire l'image en mouvement indexée endoscopique et l'image en mouvement indexée ultrasonore sur l'affichage.

2. Système de traitement d'images médicales selon la revendication 1,
dans lequel le processeur est configuré pour :
lire simultanément le signal vidéo endoscopique et le signal vidéo ultrasonore soumis au processus de synchronisation sur l'affichage.

3. Système de traitement d'images médicales selon la revendication 2,
dans lequel le signal vidéo endoscopique et le signal vidéo ultrasonore sont lus automatiquement ou par une opération d'un utilisateur.

4. Système de traitement d'images médicales selon la revendication 1,
dans lequel le processeur est configuré pour :
détecter un instant de non-déglutition à partir du signal vidéo ultrasonore ;
identifier le signal vidéo endoscopique ayant le même temps d'imagerie que le signal vidéo ultrasonore à un instant autre que l'instant de non-déglutition parmi les signaux vidéo endoscopiques à l'instant de déglutition et extraire une image en mouvement indexée endoscopique ;
identifier le signal vidéo ultrasonore ayant le même temps d'imagerie que le signal vidéo endoscopique à l'instant de déglutition parmi les signaux vidéo ultrasonores à l'instant autre que l'instant de non-déglutition et extraire une image en mouvement indexée ultrasonore.

5. Système de traitement d'images médicales selon la revendication 4,
dans lequel l'image en mouvement indexée endoscopique (44) et l'image en mouvement indexée ultrasonore (54) sont lues automatiquement ou par une opération d'un utilisateur.

6. Système de traitement d'images médicales selon la revendication 4 ou la revendication 5,
dans lequel le processeur est configuré pour détecter l'un quelconque de déglutition normale, d'aspiration ou de déglutition inconnue comme instant de déglutition à partir du signal vidéo endoscopique et du signal vidéo ultrasonore.

7. Système de traitement d'images médicales selon l'une quelconque des revendications 4 à 6,
dans lequel le processeur est configuré pour déterminer si l'instant de déglutition est présent ou non dans le signal vidéo endoscopique, en utilisant l'un quelconque de calcul d'une quantité de flou entre les images de trame, de calcul du nombre de points clés sur la base de l'image de trame, ou d'une différence entre des valeurs de pixels des images de trame, et déterminer si l'instant de déglutition est présent ou non dans le signal vidéo ultrasonore en utilisant l'apprentissage automatique.

8. Système de traitement d'images médicales selon l'une quelconque des revendications 4 à 7,
dans lequel le processeur est configuré pour analyser au moins l'une de l'image en mouvement indexée endoscopique ou de l'image en mouvement indexée ultrasonore pour spécifier un type de l'examen de déglutition.

9. Système de traitement d'images médicales selon la revendication 8,
dans lequel le processeur est configuré pour classer les types des examens de déglutition en fonction d'un type d'aliment d'examen de déglutition et d'un résultat de déglutition et attribuer des numéros d'index à l'image en mouvement indexée endoscopique et à l'image en mouvement indexée ultrasonore sur la base de la classification.

10. Système de traitement d'images médicales selon l'une quelconque des revendications 1 à 9,
dans lequel le processeur est configuré pour effectuer une imagerie à une fréquence d'images de 60 images par seconde (frames per second, fps) lors de l'acquisition du signal vidéo endoscopique et du signal vidéo ultrasonore, et
le temps est compté en centisecondes lors de l'imagerie.
